(19) Europäisches Patentamt
European Patent Office
Office européen des brevets

(11) EP 1 304 099 A2

(12) **DEMANDE DE BREVET EUROPEEN**

(43) Date de publication:
**23.04.2003 Bulletin 2003/17**

(51) Int Cl.[7]: **A61K 7/02**

(21) Numéro de dépôt: **02292422.9**

(22) Date de dépôt: **02.10.2002**

(84) Etats contractants désignés:
**AT BE BG CH CY CZ DE DK EE ES FI FR GB GR IE IT LI LU MC NL PT SE SK TR**
Etats d'extension désignés:
**AL LT LV MK RO SI**

(30) Priorité: **17.10.2001 FR 0113380**

(71) Demandeur: **L'OREAL**
**75008 Paris (FR)**

(72) Inventeurs:
- **Auguste, Frédéric**
**94550 Chevilly-Larue (FR)**
- **De La Poterie, Valérie**
**77820 Le Chatelet en Brie (FR)**
- **Portois, Emmanuelle**
**92320 Chatillon (FR)**

(74) Mandataire: **Boulard, Denis**
**L'OREAL-DPI**
**6 rue Bertrand Sincholle**
**92585 Clichy Cédex (FR)**

(54) **Composition cosmétique filmogène**

(57) L'invention a pour objet l'utilisation d'un polymère filmogène hydrophobe et d'un tensioactif ionique présentant une variation de conductivité dans l'eau, à une concentration de 2 % en poids dans l'eau, d'au moins 100 µS/cm dans une gamme de température allant de 20 °C à 45 °C, dans une composition comprenant un milieu physiologiquement acceptable pour obtenir un film déposé sur les matières kératiniques démaquillable à l'eau chaude.

Application au maquillage et au soin des matières kératiniques.

EP 1 304 099 A2

## Description

La présente invention a pour objet l'utilisation d'un polymère filmogène hydrophobe et d'un tensioactif particulier pour l'obtention d'un film démaquillable à l'eau chaude.
L'invention a aussi pour objet une composition de soin ou de maquillage des matières kératiniques comme la peau, les cils, les sourcils, les cheveux et les ongles, notamment d'êtres humains comprenant un un polymère filmogène hydrophobe et un tensioactif particulier, ainsi qu'un procédé de maquillage ou de soin cosmétique des matières kératiniques.

La composition peut se présenter sous la forme de mascara, d'eye-liner, de produit pour les lèvres, de fard à joues ou à paupières, de fond de teint, de produit de maquillage du corps, de produit anti-cernes, de produit pour les ongles, de composition de protection solaire, de coloration de la peau, de produit de soin de la peau. Plus spécialement, l'invention porte sur un mascara.

Il est connu du document WO-A-95/15741 des compositions de mascaras sous forme d'émulsion cire-dans-eau comprenant des tensio-actifs. Toutefois, le film de maquillage obtenu avec ces compositions ne présente pas une bonne résistance à l'eau et le film au contact de l'eau, pendant la baignade ou la douche par exemple, se désagrège en partie en s'effritant ou bien encore en s'étalant autour de l'oeil. L'effritement du film engendre une perte sensible de l'intensité de la couleur du maquillage, obligeant ainsi la consommatrice à renouveler l'application du mascara. L'étalement du film forme, quant à lui, une auréole autour de la zone maquillée très inesthétique. Les larmes et la transpiration provoquent également ces mêmes inconvénients.

Pour favoriser la tenue à l'eau du maquillage, il est connu du document US-A-4423031 d'utiliser des polymères acryliques en dispersion aqueuse. Néanmoins, le mascara est difficile à démaquiller et nécessite l'emploi de démaquillants spécifiques à base d'huiles ou de solvants organiques. Or ces démaquillants peuvent être irritants pour l'oeil, notamment provoquer des picotements ou laisser un voile sur l'oeil, ou bien encore peuvent laisser sur la peau autour de l'oeil (paupières) un film résiduel gras inconfortable.

Pour éviter l'emploi de ces démaquillants spécifiques, il est possible d'employer de l'eau et du savon comme le décrit le document WO-A-96/33690 en proposant un mascara comprenant un polymère insoluble dans l'eau et un polymère filmogène hydrosoluble. Toutefois, l'emploi de savon peut provoquer un inconfort occulaire dû à des picotements ou au dépôt d'un voile sur l'oeil. Le savon solubilise aussi le film de maquillage qui s'étale alors autour de l'oeil et forme des auréoles inesthétiques et tache la peau.

L'emploi d'eau chaude, c'est-à-dire d'eau ayant une température supérieure ou égale à 35 °C (température mesurée à la pression atmosphérique), et notamment allant environ de 35 °C à 50 °C, permet d'éviter les inconvénients des démaquillants connus jusqu'à présents mais les compositions de mascara résistante à l'eau froide décrites précédemment ne sont pas éliminables à l'eau chaude.

Le but de la présente invention est donc de proposer une composition cosmétique démaquillable à l'eau chaude tout en présentant une bonne tenue à l'eau froide.

Les inventeurs ont découvert qu'une telle composition pouvait être obtenue en utilisant un polymère filmogène apte à former un film hydrophobe et un tensioactif particulier. Après application de la composition sur les matières kératiniques, notament sur les cils, le maquillage obtenu est bien résistant à l'eau froide, c'est-à-dire à une eau ayant une température inférieure ou égale à 30 °C, lors de baignade par exemple, et/ou aux larmes et/ou à la transpiration.
Le maquillage s'élimine facilement avec de l'eau chaude, notamment par frottements avec un coton ou une gaze : le maquillage se décolle facilement des cils et est retiré des cils sans se fragmenter (en forme de gaine) ou sous forme de fragments ou de morceaux. Le maquillage ainsi éliminé ne s'étale pas sur la peau ce qui évite la formation d'auréoles autour de l'oeil ; la peau n'est pas tachée lors du démaquillage et reste propre. Le maquillage s'élimine très simplement avec de l'eau chaude et en particulier avec de l'eau chaude ne contenant pas d'agent détergent tel que les savons.
Pour le démaquillage, l'eau chaude utilisée peut être de l'eau de robinet, de l'eau déminéralisée ou bien encore de l'eau minérale portées à une température supérieure ou égale à 35 °C, et notamment allant environ de 35 °C à 50 °C.

De façon plus précise, l'invention a pour objet l'utilisation d'un polymère filmogène hydrophobe et d'un tensioactif ionique présentant une variation de conductivité dans l'eau, à une concentration de 2 % en poids dans l'eau, d'au moins 100 µS/cm dans une gamme de température allant de 20 °C à 45 °C, dans une composition comprenant un milieu physiologiquement acceptable, pour obtenir un film déposé sur les matières kératiniques démaquillable à l'eau chaude.

Par milieu physiologiquement acceptable, il faut comprendre un milieu compatible avec les matières kératiniques, comme un milieu cosmétique.

Avantageusement, la composition selon l'invention contient peu, voire est exempte, de tensioactif additionnel différent dudit tensioactif ionique défini précédemment, notamment en une teneur inférieure à 0,5 % en poids, par rapport au poids total de la composition. La composition présente ainsi une bonne tenue à l'eau froide.

On entend par tensioactif additionnel tout composé amphiphile choisi parmi les composés amphiphiles non-ioniques ayant un HLB (hydrophile-lipophile balance) supérieur ou égale à 10 et les composés amphiphiles ioniques

dont la partie hydrophile comprend un contre-ion ayant une masse molaire supérieure ou égale à 50 g/mole, ces composés amphiphiles ioniques étant différents dudit tensioactif ionique défini précédemment.

Le démaquillage à l'eau chaude du film est obtenu en utilisant un tensioactif ionique présentant une variation de conductivité dans l'eau, à une concentration de 2 % en poids dans l'eau, d'au moins 100 µS/cm dans une gamme de température allant de 20 °C à 45 °C, avantageusement allant de 30 °C à 45 °C, et de préférence allant de 40 °C à 45 °C. En particulier, ladite variation de conductivité va de 100 à 2000 µS/cm, de préférence va de 200 à 1500 µS/cm, et mieux va de 200 à 1000 µS/cm.

Ce tensioactif ionique particulier rend le film de polymère plus sensible à l'eau : le film de maquillage est fragilisé lors du contact avec l'eau chaude et en le frottant, par exemple avec les doigts ou bien avec un tissu ou un coton, le film se désagrège facilement ou se décolle de son support.

Ledit tensioactif ionique est de préférence un tensioactif anionique. Il peut être choisi parmi le stéarate de sodium, le stéarate de potassium, et les phtalamates de formule (I) :

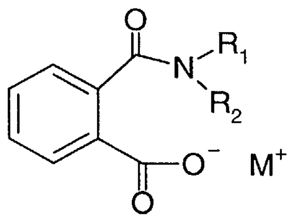

(I)

dans laquelle $R_1$ désigne un radical alkyle ayant de 16 à 20 atomes de carbone, $R_2$ désigne H ou $-CH_3$, M désigne H, Na, K, $NH_4$, $(CH_3CH_2)_3NH$, $(CH_3CH_2)_2NH_2$, $(HOCH_2CH_2)NH$, $(HOCH_2CH_2)NH_2$ . De préférence $R_1$ désigne un radical alkyle ayant 18 atomes de carbone; $R_2$ désigne un atome d'hydrogène.

Ledit tensioactif ionique est de préférence, ledit tensioactif ionique est choisi parmi le stéarate de sodium et le N-octadécyl phtalamate de sodium et plus particulièrement est le N-octadécyl phtalamate de sodium.

Avantageusement, ledit tensioactif ionique a un poids moléculaire allant de 200 à 1000, de préférence allant de 250 à 800, et préférentiellement allant de 250 à 600. (stérate de sodium = 306 ; N-octadécyl phtalamate de sodium = 440)

Aussi, l'invention a pour objet une composition comprenant, dans un milieu physiologiquement acceptable, au moins un polymère filmogène hydrophobe et au moins un tensioactif ionique de formule (I) :

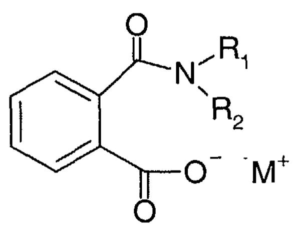

(I)

dans laquelle $R_1$ désigne un radical alkyle ayant de 16 à 20 atomes de carbone, $R_2$ désigne H ou $-CH_3$, M désigne H, Na, K, $NH_4$, $(CH_3CH_2)_3NH$, $(CH_3CH_2)_2NH_2$, $(HOCH_2CH_2)NH$, $(HOCH_2CH_2)NH_2$ . De préférence $R_1$ désigne un radical alkyle ayant 18 atomes de carbone; $R_2$ désigne un atome d'hydrogène.

Ledit tensioactif anionique peut être présent dans la composition en une teneur allant de 0,1 % à 20 % en poids, par rapport au poids total de la composition, de préférence allant de 0,5 % à 15 % en poids, et mieux allant de 1 % à 10 % en poids.

Avantageusement, la composition peut comprendre en outre du monostérate de glycérol, notamment en une teneur allant de 0,1 % à 5 % en poids, par rapport au poids total de la composition, et de préférence allant de 0,5 % à 2 % en poids. Ce composé associé au tensioactif ionique permet d'obtenir une variation de la conductivité décrite prédemment pour ce mélange beaucoup plus importante que celle dudit tensioactif ionique seul et ainsi obtenir un démaquillage à l'eau chaude du film déposé sur les matières kératiniques encore plus facile.

De préférence, ledit tensioactif ionique et le monostéarate de glycérol peuvent être présents dans la composition selon un rapport pondéral tensioactif ionique / monostéarate de glycérol allant de 0,01 à 2, de préférence de 0,5 à 2, et mieux de 1 à 2.

Selon l'invention, la composition comprend également un polymère filmogène apte à former un film hydrophobe. Dans la présente demande, on entend par "polymère filmogène" un polymère apte à former à lui seul ou en présence d'agent auxiliaire de filmification, un film continu et adhérent sur un support, notamment sur les matières kératiniques.

Par "polymère filmogène apte à former un film hydrophobe", on entend un polymère dont le film a une solubilité dans l'eau à 25 °C inférieure à 1 % en poids.

Le polymère filmogène peut être choisi parmi les polymères synthétiques, notamment les polymères radicalaires ou les polycondensats, les polymères d'origine naturelle, et leurs mélanges.

Par polymère filmogène radicalaire, on entend un polymère obtenu par polymérisation de monomères à insaturation notamment éthylénique (à l'inverse des polycondensats).
Les polymères filmogènes de type radicalaires peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, qui peut être linéaire, ramifié ou cyclique, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le (méth)acrylate de cyclo-hexyle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_1$-$C_{20}$. Parmi les N-alkyl (méth)acrylamides, on peut citer le N-éthyl acrylamide, le N-t-butyl acrylamide et le N-t-octyl acrylamide, le N-undécyl acrylamide.

Les polymères filmogènes vinyliques peuvent également résulter de l'homopolymérisation ou de la copolymérisation d'au moins un monomère choisi parmi les esters vinyliques, les oléfines (y compris les oléfines fluorées), les éthers vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Parmi les oléfines, on peut citer l'éthylène, le propylène, le butène, l'isobutène, l'octène, l'octadécène, les polyoléfines fluorées comme le tétrafluoroéthylène, le fluorure de vinylidène, l'hexafluoropropène, le chlorotrifluoroéthylène.
Comme monomères styrèniques, on peut citer le styrène et l'alpha-méthyl styrène.

Comme polycondensats utilisables comme polymère filmogène, on peut employer les polyuréthanes, notamment les polyuréthanes anioniques, cationiques, non-ioniques ou amphotères, les polyuréthanes-acryliques, les polyuréthanes-polyvinylpirrolidones, les polyester-polyuréthanes, les polyéther-polyuréthanes, les polyurées, les polyurée/polyuréthanes, et leurs mélanges.
Le polyuréthane filmogène peut être, par exemple, un copolymère polyuréthane, polyurée/uréthane, ou polyurée, aliphatique, cyclique ou aromatique, comportant seule ou en mélange :

- au moins une séquence d'origine polyester aliphatique et/ou cyclique et/ou aromatique, et/ou,
- au moins une séquence siliconée, ramifiée ou non, par exemple polydiméthylsiloxane ou polyméthylphénylsiloxane, et/ou
- au moins une séquence comportant des groupes fluorés.

Parmi les polycondensats filmogènes, on peut également citer les polyesters, les polyesters amides, les polyesters à chaîne grasse, les polyamides, et les résines époxyesters, les résines résultant de la condensation de formaldéhyde avec une arylsulfonamide, les résines aryl-sulfonamide époxy.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide diglycolique, l'acide thiodipropionique, l'acide 2,5-naphtalènedicarboxylique, l'acide 2,6-naphta-lènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.
Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol.
Comme autres polyols, on peut utiliser le glycérol, le pentaérythritol, le sorbitol, le triméthylol propane.
Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.
Le polyester peut en outre comprendre au moins un monomère portant au moins un groupement $-SO_3M$, avec M représentant un atome d'hydrogène, un ion ammonium $NH_4^+$ ou un ion métallique, comme par exemple un ion $Na^+$, $Li^+$, K+, $Mg^{2+}$, $Ca^{2+}$, $Cu^{2+}$, $Fe^{2+}$, $Fe^{3+}$. On peut utiliser notamment un monomère aromatique bifonctionnel comportant un tel groupement $-SO_3M$.
Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement $-SO_3M$ : l'acide sulfoisophtalique, l'acide sulfotéréphtalique, l'acide sulfophtalique, l'acide 4-sulfonaphtalène-2,7-dicarboxylique.
On préfère utiliser dans les compositions objet de l'invention des copolymères à base d'isophtalate/sulfoisophtalate, et plus particulièrement des copolymères obtenus par condensation de di-éthylèneglycol, cyclohexane diméthanol, acide isophtalique, acide sulfoisophtalique. De tels polymères sont vendus par exemple sous le nom de marque Eastman AQ par la société Eastman Chemical Products.
Le polymère hydrophobe synthétique peut également être un polymère siliconé, par exemple de type polyorganopolysiloxane.
Les polymères d'origine naturelle, éventuellement modifiés, peuvent être choisis parmi la résine shellac, la gomme de sandaraque, les dammars, les élémis, les copals, les polymères cellulosiques tels que la nitrocellulose, l'acétate de cellulose, l'acétobutyrate de cellulose, l'acétopropionate de cellulose, l'éthyl cellulose, et leurs mélanges.
Selon un première variante de réalisation de l'invention, le polymère peut être présent sous forme de particules solides dispersées dans un milieu aqueux. Par polymère sous forme de particules en dispersion aqueuse, connu généralement sous le nom de latex ou pseudolatex, on entend une phase contenant de l'eau et éventuellement un composé soluble dans l'eau, dans laquelle est dispersé directement le polymère sous forme de particules.
La taille des particules de polymères en dispersion aqueuse peut aller de 10 nm à 500 nm, et de préférence de 20 nm à 300 nm.
Le milieu aqueux peut être constitué essentiellement d'eau ou bien encore comprendre également un mélange d'eau et de solvant miscible à l'eau comme les monoalcools inférieurs ayant de 1 à 5 atomes de carbone, les glycols ayant de 2 à 8 atomes de carbone, les cétones en $C_3$-$C_4$, les aldéhydes en $C_2$-$C_4$. Il représente, en pratique, de 5 % à 94,9 % en poids, par rapport au poids total de la composition.
Comme polymère filmogène en dispersion aqueuse, on peut utiliser les polymères acryliques vendus sous les dénominations NEOCRYL XK-90®, NEOCRYL A-1070®, NEOCRYL A-1090®, NEOCRYL BT-62®, NEOCRYL A-1079®, NEOCRYL A-523® par la société AVECIA-NEORESINS, DOW LATEX 432® par la société DOW CHEMICAL, ou bien encore les polyuréthanes tels que les polyester-polyuréthanes vendus sous les dénominations "AVALURE UR-405®", "AVALURE UR-410®", "AVALURE UR-425®", "SANCURE 2060®" par la société GOODRICH, les polyéther-polyuréthanes vendus sous les dénominations "SANCURE 878®", "AVALURE UR-450®" par la société GOODRICH, "NEOREZ R 970®" par la société ICI, les polyuréthanes-acrylique vendus sous la dénomination NEOREZ R-989® par la société AVECIA-NEORESINS.
Il est également possible d'utiliser des polymères dits alcali-solubles en veillant à ce que le pH de la composition soit ajusté pour maintenir ces polymères à l'état de particules en dispersion aqueuse.
La composition selon l'invention peut comprendre un agent auxiliaire de filmification favorisant la formation d'un film avec les particules du polymère filmogène. Un tel agent de filmification peut être choisi parmi tous les composés connus de l'homme du métier comme étant susceptibles de remplir la fonction recherchée, et notamment être

choisi parmi les agents plastifiants et les agents de coalescence.

Selon une deuxième variante de réalisation de l'invention, le polymère filmogène peut être présent sous forme de particules stabilisées en surface et dispersées dans une phase grasse liquide.

De préférence, la phase grasse liquide comprend une phase grasse liquide volatile, éventuellement en mélange avec une phase grasse liquide non volatile.

Par "phase grasse volatile", on entend tout milieu non aqueux susceptible de s'évaporer de la peau en moins d'une heure. Cette phase volatile comporte notamment des huiles ayant une pression de vapeur, à température ambiante et pression atmosphérique allant de $10^{-3}$ à 300 mm de Hg (0,13 Pa à 40 000 Pa).

La phase grasse liquide dans laquelle est dispersé le polymère, peut être constituée de toute huile physiologiquement acceptable et en particulier cosmétiquement acceptable, notamment choisie parmi les huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée.

La phase grasse liquide totale de la composition peut représenter de 5 % à 98 % en poids, par rapport au poids total de la composition et de préférence de 20 à 85 % en poids. La partie non volatile peut représenter de 0 à 80 % (notamment de 0,1 à 80 %) du poids total de la composition et mieux de 1 à 50%.

Comme phase grasse liquide utilisable dans l'invention, on peut ainsi citer les esters d'acide gras, les acides gras supérieurs, les alcools gras supérieurs, les polydiméthylsiloxane (PDMS), éventuellement phénylées telles que les phényltriméthicones, ou éventuellement substitués par des groupements aliphatiques et/ou aromatiques, éventuellement fluorés, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées.

Avantageusement, on peut utiliser une ou plusieurs huiles volatiles à température ambiante. Après évaporation de ces huiles, on obtient un dépôt filmogène souple, non collant. Ces huiles volatiles facilitent, en outre, l'application de la composition sur les fibres kératiniques comme les cils.

Ces huiles volatiles peuvent être des huiles hydrocarbonées, des huiles siliconées comportant éventuellement des groupements alkyle ou alkoxy en bout de chaîne siliconée ou pendante.

Comme huile de silicone volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone. on peut citer notamment l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane.

Comme huile hydrocarbonée volatile, on peut citer les isoparaffines en $C_8$-$C_{16}$ telles que les 'ISOPARs', les PERMETYLs et notamment l'isododécane.

Ces huiles volatiles peuvent être présentes dans la composition en une teneur allant de 5 à 94,9 % du poids total de la composition, et mieux de 20 à 85 %.

Dans un mode particulier de réalisation de l'invention, on choisit la phase grasse liquide dans le groupe comprenant :

- les composés liquides non aqueux ayant un paramètre de solubilité global selon l'espace de solubilité de HANSEN inférieur à 17 $(MPa)^{1/2}$,
- ou les monoalcools ayant un paramètre de solubilité global selon l'espace de solubilité de HANSEN inférieur ou égal à 20 $(MPa)^{1/2}$,
- ou leurs mélanges.

Le paramètre de solubilité global $\delta$ global selon l'espace de solubilité de HANSEN est défini dans l'article "Solubility parameter values" de Eric A. Grulke de l'ouvrage "Polymer Handbook" 3ème édition, Chapitre VII, pages 519-559 par la relation :

$$\delta = (d_D{}^2 + d_P{}^2 + d_H{}^2)^{1/2}$$

dans laquelle

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc.). La définition des solvants dans l'espace de solubilité tridimensionnel selon HANSEN est décrite dans l'article de

C. M. HANSEN : "The three dimensional solubility parameters" J. Paint Technol. 39, 105 (1967).

Des huiles pouvant être utilisées dans la phase grasse liquides sont par exemples citées dans la demande EP-A-749747. Comme milieu non aqueux, on peut aussi utiliser ceux décrits dans le document FR-A-2 710 646 de L. V.M.H.

Le choix du milieu non aqueux est effectué par l'homme du métier en fonction de la nature des monomères constituant le polymère et/ou de la nature du stabilisant, comme indiqué ci-après.

La dispersion de polymère peut être fabriquée comme décrit dans le document EP-A-749747. La polymérisation peut être effectuée en dispersion, c'est-à-dire par précipitation du polymère en cours de formation, avec protection des particules formées avec un stabilisant.

Les particules de polymère en dispersion dans ladite phase grasse ont de préférence une taille allant de 5 nm à 600 nm, et de préférence de 50 nm à 250 nm.

Les particules de polymère sont stabilisées en surface grâce à un stabilisant qui peut être un polymère séquencé, un polymère greffé, et/ou un polymère statistique, seul ou en mélange.

Parmi les polymères greffés, on peut citer les polymères siliconés greffés avec une chaîne hydrocarbonée ; les polymères hydrocarbonés greffés avec une chaîne siliconée.

Conviennent également les copolymères greffés ayant par exemple un squelette insoluble de type polyacrylique avec des greffons solubles de type acide poly (12-hydroxy stéarique).

On peut également utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.

Le stabilisant peut aussi être choisi parmi les copolymères blocs greffés ou séquencés comprenant au moins un bloc de type polyorganosiloxane et au moins d'un polyéther. Le bloc polyorganopolysiloxane peut être notamment un polydiméthylsiloxane ou bien encore un poly alkyl($C_2$-$C_{18}$) méthyl siloxane ; le bloc polyéther peut être un poly alkylène en $C_2$-$C_{18}$, en particulier polyoxyéthylène et/ou polyoxypropylène. En particulier, on peut utiliser les diméthicones copolyol ou encore les alkyl ($C_2$-$C_{18}$) méthicones copolyol. On peut par exemple utiliser le diméthicone copolyol vendu sous la dénomination "DOW CORNING 3225C" par la société DOW CORNING, ou le lauryl méthicone copolyol vendu sous la dénomination "DOW CORNING Q2-5200 par la société "DOW CORNING".

Comme copolymères blocs greffés ou séquencés, on peut utiliser les copolymères comprenant au moins un bloc résultant de la polymérisation d'au moins un monomère éthylénique, à une ou plusieurs liaisons éthyléniques éventuellement conjuguées, tels que l'éthylène, le butadiène, l'isoprène, et d'au moins un bloc d'un polymère styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces copolymères séquencés, on peut citer les copolymères de type "dibloc" ou "tribloc" du type polystyrène/polyisoprène, polystyrène/polybutadiène tels que ceux vendus sous le nom de 'LUVITOL HSB' par BASF, du type polystyrène/copoly(éthylène-propylène) tels que ceux vendus sous le nom de 'KRATON' par Shell Chemical Co ou encore du type polystyrène/copoly(éthylène-butylène).

Comme copolymères blocs greffés ou séquencés comprenant au moins un bloc résultant de la polymérisation d'au moins un monomère éthylénique, tels que l'éthylène, l'isobutylène, et d'au moins un bloc d'un polymère acrylique tel que le méthacrylate de méthyle, on peut citer les copolymères bi- ou triséquencés poly(méthylacrylate de méthyle) /polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'au moins un monomère éthylénique et d'au moins un bloc d'un polyéther tel qu'un polyoxyalkylène en C2-C18, en particulier polyoxyéthylène et/ou polyoxypropylène, on peut citer les copolymères bi- ou triséquencés polyoxyéthylène/polybutadiène ou polyoxyéthylène/polyisobutylène.

On peut également employer des copolymères de (méth)acrylates d'alkyl en $C_1$-$C_4$, et de (méth)acrylates d'alkyl en $C_8$-$C_{30}$. On peut en particulier citer le copolymère méthacrylate de stéaryle/méthacrylate de méthyle.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

Lorsque la phase grasse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxy ($C_2$-$C_{18}$)alkylène et notammment polyoxypropyléné et/ou oxyéthyléné.

Lorsque la phase grasse liquide ne comprend pas d'huile de silicone, l'agent stabilisant est de préférence choisi dans le groupe constitué par :

- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alkyl en $C_1$-$C_4$, et d'acrylates ou de méthacrylates d'alkyl en $C_8$-$C_{30}$,
- (c) les copolymères blocs greffés ou séquencés comprenant au moins un bloc résultant de la polymérisation d'au moins un monomère éthylénique, à liaisons éthyléniques conjuguées,

et au moins un bloc d'un polymère vinylique ou acrylique ou d'un polyéther ou d'un polyester, ou leurs mélanges.

De préférence, on utilise des polymères dibloc comme agent stabilisant.

Selon une troisième variante de réalisation de l'invention, le deuxième polymère filmogène peut être présent sous forme solubilisé dans une phase grasse liquide telle que définie précédemment, appelé encore polymère liposoluble.

A titre d'exemple de polymère liposoluble, on peut citer les polymères correspondant à la formule (II) suivante :

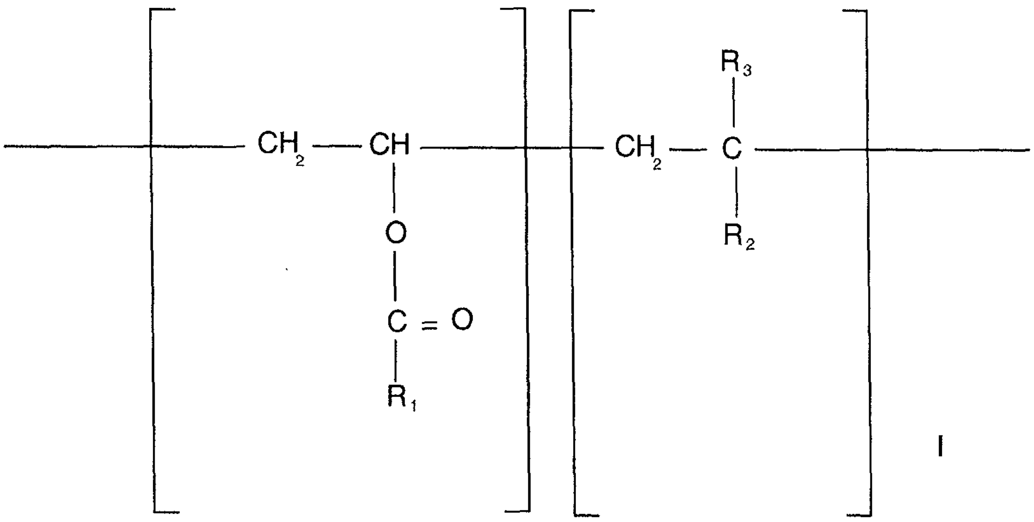

I

Ia Ib

dans laquelle :

- $R_1$ représente une chaîne hydrocarbonée saturée, linéaire ou ramifiée, ayant de 1 à 19 atomes de carbone ;
- $R_2$ représente un radical pris dans le groupe constitué par :

  a) $-O-CO-R_4$, $R_4$ ayant la même signification que $R_1$ mais est différent de $R_1$ dans un même copolymère,

  b) $-CH_2-R_5$, $R_5$ représentant une chaîne hydrocarbonée saturée, linéaire ou ramifiée, ayant de 5 à 25 atomes de carbone,

  c) $-O-R_6$, $R_6$ représentant une chaîne hydrocarbonée saturée, ayant de 2 à 18 atomes de carbone, et

  d) $-CH_2-O-CO-R_7$, $R_7$ représentant une chaîne hydrocarbonée saturée, linéaire ou ramifiée, ayant de 1 à 19 atomes de carbone,

- $R_3$ représente un atome d'hydrogène quand $R_2$ représente les radicaux a), b) ou c) ou $R_3$ représente un radical méthyle quand $R_2$ représente le radical d), ledit copolymère devant être constitué d'au moins 15 % en poids d'au moins un monomère dérivé d'un motif (Ia) ou d'un motif (Ib) dans lesquels les chaînes hydrocarbonées, saturées ou ramifiées, ont au moins 7 atomes de carbone.

Les copolymères de formule (I) résultent de la copolymérisation d'au moins un ester vinylique (correspondant au motif Ia) et d'au moins un autre monomère (correspondant au motif Ib) qui peut être une α-oléfine, un alkylvinyléther ou un ester allylique ou méthalylique.

Lorsque dans le motif (Ib) $R_2$ est choisi parmi les radicaux $-CH_2-R_5$, $-O-R_6$ ou $-CH_2-O-CO-R_7$ tels que définis précédemment, le copolymère de formule (I) peut être constitué de 50 à 95 % en moles d'au moins un motif (la) et de 5 à 50 % en moles d'au moins un motif (Ib).

Les copolymères de formule (I) peuvent également résulter de la copolymérisation d'au moins un ester vinylique et d'au moins un autre ester vinylique différent du premier. Dans ce cas, ces copolymères peuvent être constitués de 10 à 90 % en moles d'au moins un motif (la) et de 10 à 90% en moles d'au moins un motif (Ib) dans lequel $R_2$ représente le radical $-O-CO-R_4$.

Parmi les esters vinyliques conduisant au motif de formule (la), ou au motif de formule (Ib) dans lequel $R_2$ = $-O-CO-R_4$, on peut citer l'acétate de vinyle, le propionate de vinyle, le butanoate de vinyle, l'octanoate de vinyle, le décanoate de vinyle, le laurate de vinyle, le stéarate de vinyle, l'isostéarate de vinyle, le diméthyl-2, 2 octanoate de vinyle, et le diméthylpropionate de vinyle.

Parmi les $\alpha$-oléfines conduisant au motif de formule (Ib) dans lequel $R_2 = -CH_2-R_5$, on peut citer l'octène-1, le dodécène-1, l'octadécène-1, l'eicosène-1, et les mélanges d' $\alpha$-oléfines ayant de 22 à 28 atomes de carbone.

Parmi les alkylvinyléthers conduisant au motif de formule (Ib) dans lequel $R_2 = -O-R_6$, on peut citer l'éthylvinyléther, le n-butylvinyléther, l'isobutylvinyléther, le décylvinyléther, le dodécylvinyléther, le cétylvinyléther et l'octadécylvinyléther.

Parmi les esters allyliques ou méthallyliques conduisant au motif de formule (Ib) dans lequel $R_2 = -CH_2-O-CO-R_7$, on peut citer les acétates, les propionates, les diméthylpropionates, les butyrates, les hexanoates, les octanoates, les décanoates, les laurates, les diméthyl-2, 2 pentanoates, les stéarates et les eicosanoates d'allyle et de méthallyle.

Les copolymères de formule (I) peuvent également être réticulés à l'aide de certains types de réticulants qui ont pour but d'augmenter sensiblement leur poids moléculaire.

Cette réticulation est effectuée lors de la copolymérisation et les réticulants peuvent être soit du type vinylique, soit du type allylique ou méthallylique. Parmi ceux-ci, on peut citer en particulier le tétraallyloxyéthane, le divinylbenzène, l'octanedioate de divinyle, le dodécanedioate de divinyle, et l'octadécanedioate de divinyle.

Parmi les différents copolymères de formule (I) utilisables dans la composition selon l'invention, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 10 à 20 atomes de carbone.

De tels homopolymères liposolubles peuvent être choisis parmi le polystéarate de vinyle, le polystéarate de vinyle réticulé à l'aide de divinylbenzène, de diallyléther ou de phtalate de diallyle, le poly(méth)acrylate de stéaryle, le polylaurate de vinyle, le poly(méth)acrylate de lauryle, ces poly(méth)acrylates pouvant être réticulés à l'aide de diméthacrylate de l'éthylène glycol ou de tétraéthylène glycol.

Les copolymères et homopolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en $C_2$-$C_{20}$, différents de la cire de polyoléfine définie en a), comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acryllque/méthacrylate de lauryle.

Le polymère filmogène peut être présent en une teneur en matière sèche allant de 5 % à 60 % en poids, par rapport au poids total de la composition, de préférence de 10 % à 45 % en poids, et mieux de 15 % à 35 % en poids.

Avantageusement, ledit tensioactif ionique et le polymère filmogène hydrophobe peuvent être présents dans la composition selon un rapport pondéral polymère filmogène hydrophobe/ tensioactif ionique allant de 30 à 0,1, de

préférence de 15 à 0,5, et mieux de 8 à 1.

La composition peut également comprendre au moins une matière colorante comme les composés pulvérulents et/ou les colorants liposolubles ou hydrosolubles, par exemple à raison de 0,01 à 50 % du poids total de la composition. Les composés pulvérulents peuvent être choisis parmi les pigments et/ou les nacres habituellement utilisés dans les compositions cosmétiques. Avantageusement, les composés pulvérulents représentent de 0,1 à 25 % du poids total de la composition et mieux de 1 à 20 %.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

La composition peut également comprendre des charges qui peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de Nylon (Orgasol de chez Atochem), de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut contenir, en outre, tout additif usuellement utilisés dans de telles compositions tels que les épaississants, les conservateurs, les parfums, les filtres solaires, les agents antiradicaux-libres, les cires, les huiles, les agents hydratants, les vitamines, les protéines, les plastifiants, les sequestrants, les céramides, les agents alcalinisants ou acidifiants, les émollients.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

Un autre objet de l'invention est l'utilisation d'une composition telle que définie précédemment pour obtenir un film déposé sur les matières kératiniques résistant à l'eau froide et/ou démaquillable à l'eau chaude.

L'invention a également pour objet un procédé cosmétique de maquillage ou de soin non thérapeutique des matières kératiniques comprenant l'application sur les matières kératiniques d'une composition telle que définie précédemment.

L'invention a aussi pour objet un procédé cosmétique de démaquillage des matières kératiniques maquillées avec une composition telle que définie précédemment, comprenant au moins une étape de rinçage avec de l'eau chaude portée à une température supérieure ou égale à 35 °C des dites matières kératiniques maquillées.

L'invention est illustrée plus en détail dans les exemples suivants.

**<u>Exemples 1 et 2</u> :**

On a préparé des mascaras ayant la composition suivante :

- Polyuréthane en dispersion aqueuse vendu sous la dénomination AVALURE UR 425 par la société GOODRICH à 49 % en poids de matières actives 21 g MA
- Hydroxyéthyl cellulose 1, 9 g
- tensioactif anionique 3 g
- Oxyde de fer noir 5 g
- Propylène glycol 5 g
- Conservateurs qs
- Eau qsp 100 g

Pour chaque composition, on a étalé une couche de 300 µm d'épaisseur (avant séchage) sur une plaque de verre, on a laissé séché pendant 24 heures à 30 °C et 50 % d'humidité relative. Après séchage, on a placé la plaque dans un cristallisoir contenant de l'eau à température ambiante (TA) (20 °C) ou à 40°C puis on a placé sur le film un

barreau aimanté. On a agité le barreau à l'aide d'un agitateur magnétique et on mesure le temps (exprimé en minutes) au bout duquel le film commence à se désagréger.

On a obtenu les résultats suivants :

| **Exemple** | **1** | **2** |
|---|---|---|
| **Tensioactif ionique** | Stéarate de sodium | N-octadécyl phtalamate de sodium |
| **20 °C** | > 2 heures | > 2 heures |
| **40 °C** | 10 minutes | 16 minutes |

On constate que pour chaque composition le film est moins résistant en présence d'eau à 40 °C (eau chaude) qu'en présence d'eau à température ambiante (eau froide). Le film est donc plus facilement démaquillable à l'eau chaude et est plus résistant à l'eau froide.

**<u>Exemple 3</u> :**

On a préparé un mascara ayant la composition suivante :

- Polyuréthane en dispersion aqueuse vendu sous la dénomination AVALURE UR 425 par la société GOODRICH à 49 % en poids de matières actives 16 g MA
- Hydroxyéthyl cellulose 1, 9 g
- N-octadécyl phtalamate de sodium 0,6 g
- Cire d'abeille 6 g
- Oxyde de fer noir 5 g
- Propylène glycol 5 g
- Conservateurs qs
- Eau qsp 100 g

Dans cette composition, le N-octadécyl phtalamate de sodium peut être remplacé par le stéarate de sodium.

Ce mascara s'applique facilement sur les cils et le maquillage obtenu présente une bonne résistance à l'eau froide et se démaquille facilement avec de l'eau chaude.

**Revendications**

1. Utilisation d'un polymère filmogène hydrophobe et d'un tensioactif ionique présentant une variation de conductivité dans l'eau, à une concentration de 2 % en poids dans l'eau, d'au moins 100 µS/cm dans une gamme de température allant de 20 °C à 45 °C, dans une composition comprenant un milieu physiologiquement acceptable, pour obtenir un film déposé sur les matières kératiniques démaquillable à l'eau chaude.

2. Utilisation selon la revendication 1, **caractérisée par le fait que** tensioactif ionique présentant une variation de conductivité dans l'eau, à une concentration de 2 % en poids dans l'eau, d'au moins 100 µS/cm dans une gamme de température allant de 30 °C à 45 °C, et de préférence allant de 40 °C à 45 °C.

3. Utilisation selon la revendication 1 ou 2, **caractérisée par le fait que** la variation de conductivité va de 100 à 2000 µS/cm, de préférence de 200 à 1500 µS/cm, et préférentiellemnt de 200 à 1000 µS/cm.

4. Utilisation selon l'une quelconque des revendications précédentes, **caractérisée par le fait que** ledit tensioactif ionique est un tensioactif anionique.

5. Utilisation selon l'une quelconque des revendications précédentes, **caractérisée par le fait que** ledit tensioactif ionique est choisi parmi le stéarate de sodium, le stéarate de potassium, et les phtalamates de formule (I) :

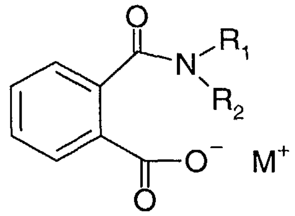

(I)

dans laquelle $R_1$ désigne un radical alkyle ayant de 16 à 20 atomes de carbone, $R_2$ désigne H ou $-CH_3$, M désigne H, Na, K, $NH_4$, $(CH_3CH_2)_3NH$, $(CH_3CH_2)_2NH_2$, $(HOCH_2CH_2)NH$, $(HOCH_2CH_2)NH_2$.

**6.** Utilisation selon la revendication 5, **caractérisée par le fait que** $R_1$ désigne un radical alkyle ayant 18 atomes de carbone.

**7.** Utilisation selon la revendication 5 ou 6, **caractérisée par le fait que** $R_2$ désigne un atome d'hydrogène.

**8.** Utilisation selon l'une quelconque des revendications précédentes, **caractérisée par le fait que** ledit tensioactif ionique est choisi parmi le stéarate de sodium et le N-octadécyl phtalamate de sodium.

**9.** Utilisation selon l'une quelconque des revendications précédentes, **caractérisée par le fait que** ledit tensioactif ionique résente une variation de conductivité dans l'eau, à une concentration de 2 % en poids dans l'eau, d'au moins 400 µS/cm dans une gamme de température allant de 20 °C à 45 °C, avantageusement allant de 30 °C à 45 °C, et de préférence allant de 40 °C à 45 °C.

**10.** Utilisation selon l'une quelconque des revendications précédentes, **caractérisée par le fait que** ledit tensioactif ionique est le N-octadécyl phtalamate de sodium.

**11.** Utilisation selon l'une quelconque des revendications précédentes, **caractérisée par le fait que** ledit tensioactif ionique a un poids moléculaire allant de 200 à 1000, de préférence allant de 250 à 800, et préférentiellement allant de 250 à 600.

**12.** Utilisation selon l'une quelconque des revendications précédentes, **caractérisée par le fait que** ledit composé organique est présent en une teneur allant de 0,1 % à 20 % en poids, par rapport au poids total de la composition, de préférence allant de 0,5 % à 15 % en poids, et mieux allant de 1 % à 10 % en poids.

**13.** Utilisation selon l'une quelconque des revendications précédentes, **caractérisée par le fait que** la composition comprend du monostéarate de glycérol.

**14.** Utilisation selon la revendication 13, **caractérisée par le fait que** le monostéarate de glycérol est présent en une teneur allant de 0,1 % à 5 % en poids, par rapport au poids total de la composition, de préférence allant de 0,5 % à 2 % en poids.

**15.** Utilisation selon l'une quelconque des revendications précédentes, **caractérisée par le fait que** ledit tensioactif ionique et le monostéarate de glycérol sont présents dans la composition selon un rapport pondéral tensioactif ionique / monostéarate de glycérol allant de 0,01 à 2, de préférence de 0,5 à 2, et mieux de 1 à 2.

**16.** Utilisation selon l'une quelconque des revendications précédentes, **caractérisée par le fait que** le polymère filmogène hydrophobe est choisi dans le groupe formé par les polymères radicalaires, les polycondensats, les polymères d'origine naturelle et leurs mélanges.

**17.** Utilisation selon l'une quelconque des revendications précédentes, **caractérisée par le fait que** le polymère filmogène hydrophobe est choisi dans le groupe formé par les polymères vinyliques, les polyuréthanes, les polyesters, les polymères cellulosiques.

**18.** Utilisation selon l'une quelconque des revendications précédentes, **caractérisée par le fait que** le polymère filmogène hydrophobe est présent sous la forme de particules dispersées dans un milieu aqueux.

**19.** Utilisation selon l'une quelconque des revendications précédentes, **caractérisée par le fait que** le polymère filmogène hydrophobe est un polyuréthane sous forme de particules en dispersion aqueuse.

**20.** Utilisation selon l'une quelconque des revendications 1 à 17, **caractérisée par le fait que** le polymère filmogène hydrophobe est présent sous la forme de particules dispersées dans une phase grasse liquide et stabilisées en surface.

**21.** Utilisation selon la revendication 20, **caractérisée par le fait que** les particules de polymère filmogène hydrophobe sont stabilisées par un stabilisant choisi parmi les polymères séquencés, les polymères greffés, les polymères statistiques et leurs mélanges.

**22.** Utilisation selon la revendication 21, **caractérisée en ce que** le stabilisant est un polymère bloc greffé ou séquencé, comprenant au moins un bloc résultant de la polymérisation d'au moins un monomère éthylénique comportant une ou plusieurs liaisons éthylénique éventuellement conjuguées et au moins un bloc d'un polymère styrénique.

**23.** Utilisation selon l'une quelconque des revendications précédentes, **caractérisée par le fait que** le polymère filmogène hydrophobe est présent en une teneur en matière sèche allant de 5 % à 60 % en poids, par rapport au poids total de la composition, de préférence de 10 % à 45 % en poids.

**24.** Utilisation selon l'une quelconque des revendications précédentes, **caractérisée par le fait que** le polymère filmogène hydrophobe et ledit tensioactif ionique sont présents dans la composition selon un rapport pondéral polymère filmogène hydrophobe / tensioactif ionique allant de 0,1 à 30, de préférence de 0,5 à 15, et mieux de 1 à 8.

**25.** Utilisation selon l'une quelconque des revendications précédentes, **caractérisée par le fait que** la composition comprend un additif cosmétique choisi parmi les matières colorantes, les charges, les épaississants, les conservateurs, les parfums, les filtres solaires, les agents antiradicaux-libres, les cires, les huiles, les agents hydratants, les vitamines, les protéines, les plastifiants, les sequestrants, les céramides, les agents alcalinisants ou acidifiants, les émollients.

**26.** Utilisation selon l'une quelconque des revendications précédentes, **caractérisée par le fait que** la composition se présente sous la forme de mascara, d'eyeliner, de produit pour les lèvres, de fard à joues ou à paupières, de fond de teint, de produit de maquillage du corps, de produit anti-cernes, de produit pour les ongles, de composition de protection solaire, de coloration de la peau, de produit de soin de la peau.

**27.** Utilisation selon l'une quelconque des revendications précédentes, **caractérisée par le fait que** la composition est un mascara.

**28.** Composition cosmétique comprenant, dans un milieu cosmétiquement acceptable, au moins un polymère filmogène hydrophobe et au moins un tensioactif ionique de formule (I) :

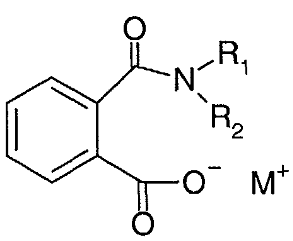

(I)

dans laquelle $R_1$ désigne un radical alkyle ayant de 16 à 20 atomes de carbone, $R_2$ désigne H ou $-CH_3$, M désigne H, Na, K, $NH_4$, $(CH_3CH_2)_3NH$, $(CH_3CH_2)_2NH_2$, $(HOCH_2CH_2)NH$, $(HOCH_2CH_2)NH_2$.

**29.** Composition selon la revendication 28, **caractérisée par le fait que** $R_1$ désigne un radical alkyle ayant 18 atomes de carbone.

**30.** Composition selon la revendication 28 ou 29, **caractérisée par le fait que** $R_2$ désigne un atome d'hydrogène.

**31.** Composition selon l'une quelconque des revendications 28 à 30, **caractérisée par le fait que** ledit tensioactif ionique est le N-octadécyl phtalamate de sodium.

**32.** Composition selon l'une quelconque des revendications 28 à 31, **caractérisée par le fait que** ledit tensioactif ionique est présent en une teneur allant de 0,1 % à 20 % en poids, par rapport au poids total de la composition, de préférence allant de 0,5 % à 15 % en poids, et mieux allant de 1 % à 10 % en poids.

**33.** Composition selon l'une quelconque des revendications 28 à 32, **caractérisée par le fait qu'**elle comprend du monostéarate de glycérol.

**34.** Composition selon la revendication 33, **caractérisée par le fait que** le monostéarate de glycérol est présent en une teneur allant de 0,1 % à 5 % en poids, par rapport au poids total de la composition, de préférence allant de 0 ,2 % à 1 % en poids.

**35.** Composition selon l'une des revendications 33 ou 34, **caractérisée par le fait que** ledit tensioactif ionique et le monostéarate de glycérol sont présents dans la composition selon un rapport pondéral tensioactif ionique / monostéarate de glycérol allant de 0,01 à 2, de préférence de 0,5 à 2, et mieux de 1 à 2.

**36.** Composition selon l'une quelconque des revendications 28 à 35, **caractérisée par le fait que** le polymère filmogène hydrophobe est choisi dans le groupe formé par les polymères radicalaires, les polycondensats, les polymères d'origine naturelle et leurs mélanges.

**37.** Composition selon l'une quelconque des revendications 28 à 36, **caractérisée par le fait que** le polymère filmogène hydrophobe est choisi dans le groupe formé par les polymères vinyliques, les polyuréthanes, les polyesters, les polymères cellulosiques.

**38.** Composition selon l'une quelconque des revendications 28 à 37, **caractérisée par le fait que** le polymère filmogène hydrophobe est présent sous la forme de particules dispersées dans un milieu aqueux.

**39.** Composition selon l'une quelconque des revendications 28 à 38, **caractérisée par le fait que** le polymère filmogène hydrophobe est un polyuréthane sous forme de particules en dispersion aqueuse.

**40.** Composition selon l'une quelconque des revendications 28 à 39, **caractérisée par le fait que** le polymère filmogène hydrophobe est présent sous la forme de particules dispersées dans une phase grasse liquide et stabilisées en surface.

**41.** Composition selon la revendication 40, **caractérisée par le fait que** les particules de polymère filmogène hydrophobe sont stabilisées par un stabilisant choisi parmi les polymères séquencés, les polymères greffés, les polymères statistiques et leurs mélanges.

**42.** Composition selon la revendication 41, **caractérisée par le fait que** le stabilisant est un polymère bloc greffé ou séquencé, comprenant au moins un bloc résultant de la polymérisation d'au moins un monomère éthylénique comportant une ou plusieurs liaisons éthylénique éventuellement conjuguées et au moins un bloc d'un polymère styrénique.

**43.** Composition selon l'une quelconque des revendications 28 à 32, **caractérisée par le fait que** le polymère filmogène hydrophobe est présent en une teneur en matière sèche allant de 5 % à 60 % en poids, par rapport au poids total de la composition, de préférence de 10 % à 45 % en poids.

**44.** Composition selon l'une quelconque des revendications 28 à 43, **caractérisée par le fait que** le polymère filmogène hydrophobe et ledit tensioactif sont présents dans la composition selon un rapport pondéral polymère filmogène hydrophobe /tensioactif ionique allant de 0,1 à 30, de préférence de 0,5 à 20, et mieux de 1 à 8.

**45.** Composition selon l'une quelconque des revendications 28 à 44, **caractérisée par le fait qu'**elle comprend, en outre, au moins un additif choisi dans le groupe formé par les épaississants, les colorants liposolubles ou hydrosolubles, les conservateurs, les parfums, les filtres solaires, les agents antiradicaux-libres, les cires, les huiles, les agents hydratants, les vitamines, les protéines, les plastifiants, les sequestrants, les céramides, les agents alcalinisants ou acidifiants, les émollients.

**46.** Composition selon l'une quelconque des revendications 28 à 45, **caractérisée par le fait qu'**elle se présente sous la forme de mascara, d'eye-liner, de produit pour les lèvres, de fard à joues ou à paupières, de fond de teint, de produit de maquillage du corps, de produit anti-cernes, de produit pour les ongles, de composition de protection solaire, de coloration de la peau, de produit de soin de la peau.

**47.** Mascara comprenant une composition selon l'une quelconque des revendications 28 à 45.

**48.** Procédé cosmétique de maquillage ou de soin non thérapeutique des matières kératiniques comprenant l'application sur les matières kératiniques d'une composition selon l'une quelconque des revendications 28 à 46.

**49.** Utilisation d'une composition selon l'une des revendications 28 à 46 pour obtenir un film déposé sur les matières kératiniques résistant à l'eau froide et/ou démaquillable à l'eau chaude.

**50.** Procédé cosmétique de démaquillage des matières kératiniques maquillées avec une composition telle que définie selon l'une quelconque des revendications 1 à 47, comprenant au moins une étape de rinçage avec de l'eau chaude portée à une température allant de 35 °C à 50 °C desdites matières kératiniques maquillées.

**51.** Procédé selon la revendication 50, **caractérisé par le fait que** l'eau chaude ne contient pas d'agent détergent.